# EUROPEAN PATENT APPLICATION

(11) **EP 4 483 807 A1**
(43) Date of publication of application: **01.01.2025**
(21) Application number: 24185743.2
(22) Date of filing: 01.07.2024
(51) Int. Cl.: A61B 5/16, G06F 3/01, A61B 5/0205, G06F 3/0481, G06F 18/00, G06V 10/80, G06V 40/20

(54) **EYE TRACKING, PHYSIOLOGY, AND SPEECH ANALYSIS FOR INDIVIDUAL STRESS AND INDIVIDUAL ENGAGEMENT**

(30) Priority: 29.06.2023 US 202318216323
(71) Applicant: Rockwell Collins, Inc., Cedar Rapids, IA 52498 (US)
(72) Inventor: WU, Peggy, East Hartford, CT (US)
(74) Representative: Dehns

(57) **Abstract**

A team monitoring system receives data for determining user stress for each team member. A team engagement metric is determined for the entire team based on individual user stress correlated to discreet portions of a task. User stress may be determined based on arm / hand positions, gaze and pupil dynamics, and voice intonation. Individual user stress is weighted according to a task priority for that individual user at the time. The system determines a team composition based on individual user stress during a task and team engagement during the task; even where the users have not engaged as a team during the task.

## Description

### GOVERNMENT LICENSE RIGHTS

The U.S. Government has a paid-up license in this invention and the right in limited circumstances to require the patent owner to license others on reasonable terms as provided by the terms of DE-AR0001097 awarded by The United States Department of Energy.

### BACKGROUND

Geographically separated teams such as pilots and ground control, or trainees and instructors experience challenges assessing each other's level of engagement and attention when they cannot see what others are seeing or how they are reacting. Gaze and eye movements are key indicators of attention but participants do not have a common shared point of reference. Further, remote teams may not be able to assess other cues such as levels of stress.

### SUMMARY

In one aspect, there is provided a team monitoring system that receives data for determining user stress for each team member. A team engagement metric is determined for the entire team based on individual user stress correlated to discreet portions of a task. User stress may be determined based on arm / hand positions, gaze and pupil dynamics, and voice intonation.

In embodiments, individual user stress is weighted according to a task priority for that individual user at the time.

In embodiments, the system determines a team composition based on individual user stress during a task and team engagement during the task; even where the users have not engaged as a team during the task.

It is to be understood that both the foregoing general description and the following detailed description are exemplary and explanatory only and should not restrict the scope of the invention which is defined by the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
FIG. 1 shows a block diagram of a system suitable for implementing embodiments of the incentive concepts disclosed herein;
FIG. 2 shows a flowchart of an exemplary embodiment of the inventive concepts disclosed herein;
FIG. 3 shows a block diagram of a neural network according an exemplary embodiment of the inventive concepts disclosed herein.

### DETAILED DESCRIPTION

Before explaining various embodiments of the inventive concepts disclosed herein in detail, it is to be understood that the inventive concepts are not limited in their application to the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways that fall within the scope of the claims. Also, it is to be understood that the phraseology and terminology employed herein is for the purpose of description and should not be regarded as limiting.

**As** used herein a letter following a reference numeral is intended to reference an embodiment of a feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Also, while various components may be depicted as being connected directly, direct connection is not a requirement. Components may be in data communication with intervening components that are not illustrated or described.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in at least one embodiment" in the specification does not necessarily refer to the same embodiment. Embodiments of the inventive concepts disclosed may include one or more of the features expressly described or inherently present herein, or any combination or sub-combination of two or more such features.

Broadly, embodiments of the inventive concepts disclosed herein are directed to a team monitoring system that receives data for determining user stress for each team member. A team engagement metric is determined for the entire team based on individual user stress correlated to discreet portions of a task. User stress may be determined based on arm / hand positions, gaze and pupil dynamics, and voice intonation. Individual user stress is weighted according to a task priority for that individual user at the time. The system determines a team composition based on individual user stress during a task and team engagement during the task; even where the users have not engaged as a team during the task.

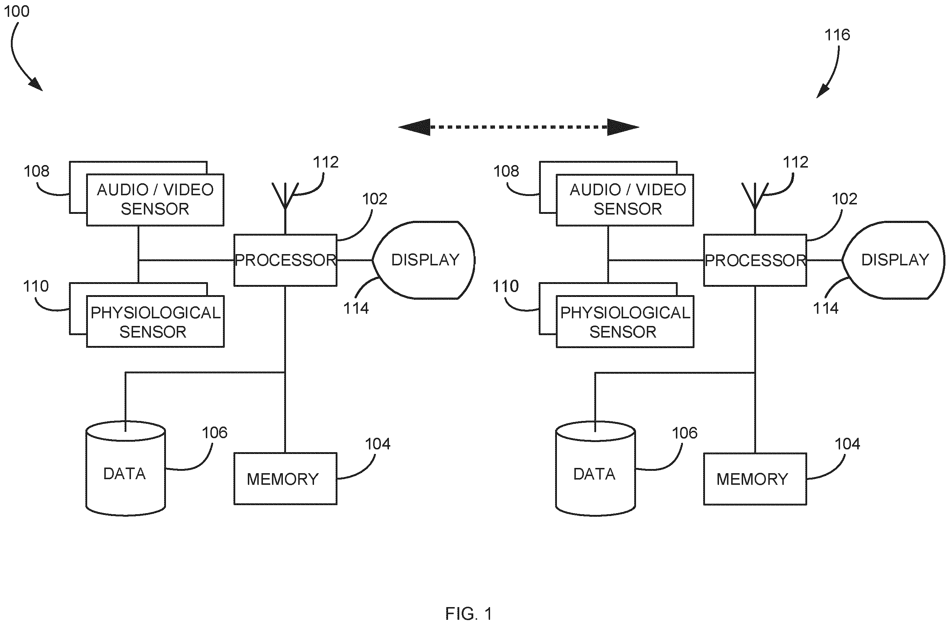

Referring to FIG. 1, a block diagram of a system suitable for implementing embodiments of the incentive concepts disclosed herein is shown. The system includes at least two nodes 100, 116, each including a processor 102, memory 104 in data communication with the processor 102 for storing processor executable code, one or more audio / video sensors 108 for receiving an audio / video data stream, and one or more physiological sensors 110. Physiological sensors 110 may include devices such as an electroencephalograph (EEG), functional near-infrared spectroscopy (fNIRs), or any other such biometric data sensing device.

In at least one embodiment, the one or more audio / video sensors 108 record eye movement / gaze of a user, eye lid position, hand / arm position and movement, other physical data landmarks, and voice intonation and volume. The processor executable code configures the processor 102 to continuously log the audio / video sensor data in a data storage element 106. The processor 102 analyzes the audio / video sensor data to identify gaze and pupil dynamics (e.g., pupil response and changes over time), and physical pose estimate for the user.

In at least one embodiment, the processor 102 records where on a display 114 certain information is rendered. Such locations may be set by the user, specific to a user profile, specific to a user's system, or the like. The processor 102 may utilize gaze and pose estimation to determine what data the user is focused on with reference to the known disposition of data on the display 114.

In at least one embodiment, the audio / video sensor data are correlated with discreet portions of a task, and / or specific stimuli such as instrument readings (including user specific locations of instrument readings on corresponding displays 114), alerts, or the like. Furthermore, the processors 102 from each node 100, 116 correlate audio /video sensor data from different users engaged in a common or collective task. Each processor 102 may receive different discreet portions of a task, specific stimuli, and alerts based on the specific function of the user; such different discreet portions, stimuli, and alerts are correlated in time such that user responses may be individually analyzed and correlated to each other to assess total team engagement. In at least one embodiment, team engagement may be weighted according to a priority of team members in time. For example, a first node 100 may analyze the stress level of a first team member performing a critical portion of the task while a second node 116 analyzes the stress level of a second team member simultaneously performing a less critical portion of the task. The assessment of total team engagement may be weighted toward the stress level of the first team member.

In at least one embodiment, total team engagement may be at least partially based on team communication. Each processor 102 may analyze speech patterns of the corresponding user to identify when the user s finished talking and gauge a response time of other team members. Furthermore, certain voice inflections / intonations may indicate a question. The processor 102 may gauge the response time of team members answering the question.

Each processor 102 may also receive physiological data from one or more corresponding physiological sensors 110. In at least one embodiment, the processor 102 may correlate audio / video sensor data (including at least gaze, pupil dynamics, and voice intonation) with physiological data. The processor 102 may compare the camera and physiological data to stored profiles. Such profiles may be specific to the user.

In at least one embodiment, analyzing speech patterns may include determining a stress metric for each user. Stress is measured with respect to voice intonation and may be specific to the user (i.e., similar voice patterns may indicate different stress levels for different users). Furthermore, stress levels may be measured with respect to the physiological sensors 110. Stress metrics for each user may be displayed to other team members and assessing team engagement may include other team member's responses to the stress metrics during verbal interaction (e.g., responsiveness to highly stressed team members, calming / escalating responses, or the like).

In at least one embodiment, the processor 102 transfers the stored audio / video sensor data and other correlated system and task data to an offline storage device for later analysis and correlation to historic data and other outside factors such as crew rest, crew sleet rhythms, flight schedules, etc. Such transfer may be in real time via the wireless communication device 112. Furthermore, team members may be correlated against each other and other team members for similar tasks to identify teams with complimentary stress patterns. For example, offline analysis may identify a team wherein no team members consistently demonstrate increased stress at the same time during similar tasks.

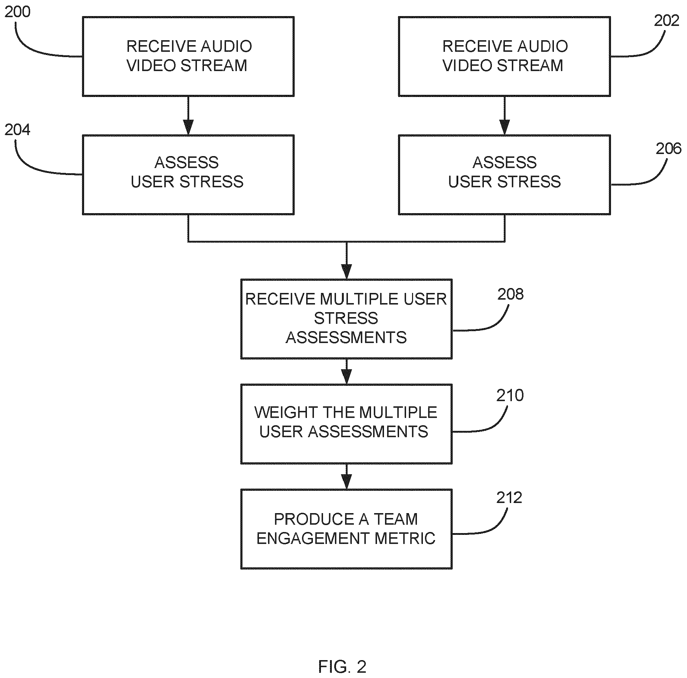

Referring to FIG. 2, a flowchart of an exemplary embodiment of the inventive concepts disclosed herein is shown. Computer systems implementing embodiments of the inventive concepts disclosed herein each receive 200, 202 an audio / video stream corresponding to one or more audio / video sensors. The audio / video stream is processed for eye tracking data (including pupil dynamics and eyelid position) and to determine physiological landmarks such as hands and arms to generate a pose estimate for the user; the audio / video stream is also processed to identify voice intonation and volume. Such data is continuously logged and used to assess 204, 206 user stress levels. For example, each computer system may compare eye gaze to predetermined expected eye gaze or scan patterns depending on a current task and a known disposition of information on a specific user display. The computer system may identify stress levels from voice intonation or volume as defined by an algorithmic model or machine learning algorithm.

In at least one embodiment, each computer system (or some separate computer system) receives 208 each user stress metric, and potentially each audio / video stream. Based on the multiple user stress metrics and audio / video streams, the computer system produces 212 a team engagement metric. The team engagement metric may be based on team member responses to user stress metrics, specifically with respect to response times to other team members. In at least one embodiment, the team engagement metric may be weighted 210 by the associated team member. For example, the computer system may define or receive a priority associated with the task of each team member and weight the corresponding stress metric by the associated priority. Alternatively, the team engagement metric may be weighted 210 according to specific stress levels of the corresponding user.

In at least one embodiment, the system receives physiological data from one or more physiological sensors such as an EEG and / or an fNIRs. Such physiological data provides the addition metric of neuroactivity when assessing 204, 206 user stress. Likewise, the system may receive data related to factors specific to the task. Such task specific data provides the additional metric of context when assessing 204, 206 user stress. Such analysis may include processing via machine learning, neural network algorithms. Tasks may define specific future actions or future action potentialities from which to make a weighted engagement assessment.

In at least one embodiment, the system may compile data to facilitate the implementation of one or more of the future actions without the intervention of the user, and potentially before the user has made a determination of what future actions will be performed. The system may prioritize data compilation based on the determined probability of each future action.

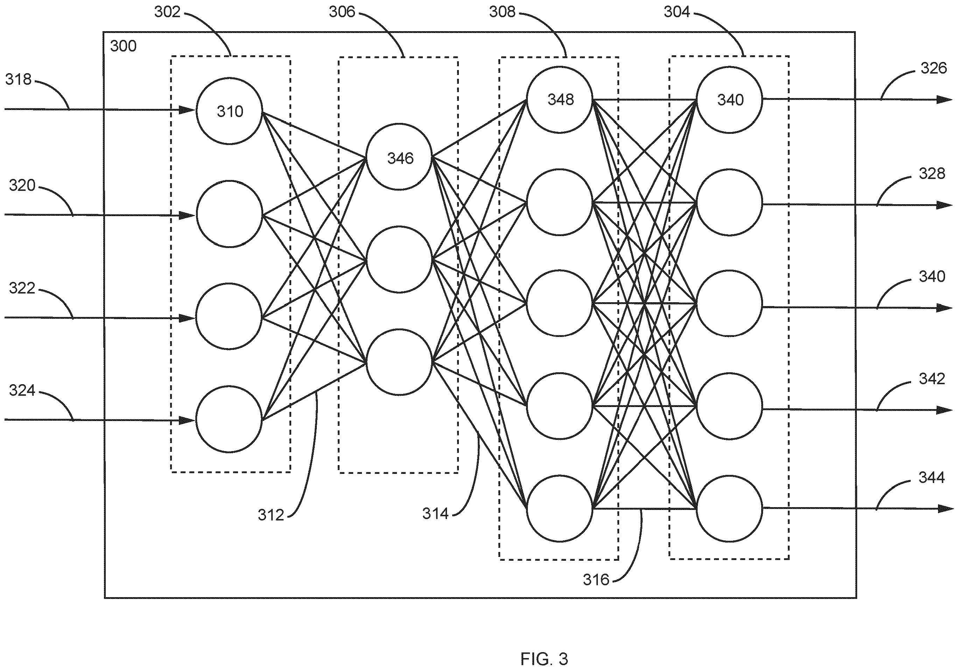

Referring to FIG. 3, a block diagram of a neural network 300 according an exemplary embodiment of the inventive concepts disclosed herein is shown. The neural network 300 comprises an input layer 302 that receives external inputs (including physiological signals, such as EEG and fNIRs, audio / video sensor data, and potentially user or task specific profiles), and output layer 304, and a plurality of internal layers 306, 308. Each layer comprises a plurality of neurons or nodes 310, 336, 338, 340. In the input layer 302, each node 310 receives one or more inputs 318, 320, 322, 324 corresponding to a digital signal and produces an output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

Outputs 312 from each of the nodes 310 in the input layer 302 are passed to each node 336 in a first intermediate layer 306. The process continues through any number of intermediate layers 306, 308 with each intermediate layer node 336, 338 having a unique set of synaptic weights corresponding to each input 312, 314 from the previous intermediate layer 306, 308. It is envisioned that certain intermediate layer nodes 336, 338 may produce a real value with a range while other intermediated layer nodes 336, 338 may produce a Boolean value. Furthermore, it is envisioned that certain intermediate layer nodes 336, 338 may utilize a weighted input summation methodology while others utilize a weighted input product methodology. It is further envisioned that synaptic weight may correspond to bit shifting of the corresponding inputs 312, 314, 316.

An output layer 304 including one or more output nodes 340 receives the outputs 316 from each of the nodes 338 in the previous intermediate layer 308. Each output node 340 produces a final output 326, 328, 330, 332, 334 via processing the previous layer inputs 316, the final output 326, 328, 330, 332, 334 corresponding to a stress metric for one or more team members. Such outputs may comprise separate components of an interleaved input signal, bits for delivery to a register, or other digital output based on an input signal and DSP algorithm. In at least one embodiment, multiple nodes may each instantiate a separate neural network 300 to process a stress metric for a single corresponding team member. Each neural network 300 may receive data from other team members as inputs 318, 320, 322, 324. Alternatively, a single neural network 300 may receive inputs 318, 320, 322, 324 from all team members, or a separate neural network 300 may receive inputs 318, 320, 322, 324 from each team member's neural network 300 to determine a team engagement metric.

In at least one embodiment, each node 310, 336, 338, 340 in any layer 302, 306, 308, 304 may include a node weight to boost the output value of that node 310, 336, 338, 340 independent of the weighting applied to the output of that node 310, 336, 338, 340 in subsequent layers 304, 306, 308. It may be appreciated that certain synaptic weights may be zero to effectively isolate a node 310, 336, 338, 340 from an input 312, 314, 316, from one or more nodes 310, 336, 338 in a previous layer, or an initial input 318, 320, 322, 324.

In at least one embodiment, the number of processing layers 302, 304, 306, 308 may be constrained at a design phase based on a desired data throughput rate. Furthermore, multiple processors and multiple processing threads may facilitate simultaneous calculations of nodes 310, 336, 338, 340 within each processing layers 302, 304, 306, 308.

Layers 302, 304, 306, 308 may be organized in a feed forward architecture where nodes 310, 336, 338, 340 only receive inputs from the previous layer 302, 304, 306 and deliver outputs only to the immediately subsequent layer 304, 306, 308, or a recurrent architecture, or some combination thereof.

Embodiments of the inventive concepts disclosed herein are critical to enabling reduced crew operations. An autonomous system can use detections of team engagement to estimate when to provide appropriate information to the users for an adaptive user interface scenario.

The ability to understand what remote team members are looking at can help teams gauge whether teammates are on task, stuck, or distracted. This can help participants assess team performance and evaluate know when to use other interventions such as task re-allocation.

It is believed that the inventive concepts disclosed herein and many of their attendant advantages will be understood by the foregoing description of embodiments of the inventive concepts, and it will be apparent that various changes may be made in the form, construction, and arrangement of the components thereof without departing from the broad scope of the inventive concepts disclosed herein or without sacrificing all of their material advantages; and individual features from various embodiments may be combined to arrive at other embodiments. The forms herein before described being merely explanatory embodiments thereof, it is the intention of the following claims to encompass and include such changes. Furthermore, any of the features disclosed in relation to any of the individual embodiments may be incorporated into any other embodiment.

## Claims

1. A computer apparatus comprising:
at least one audio / video sensor (108);
a data communication device; and
at least one processor (102) in data communication with a memory (104) storing processor executable code; and
wherein the processor executable code configures the at least one processor to:
receive an audio / video stream from the at least one audio / video sensor;
determine a user stress metric based on the audio / video stream;
receive one or more contemporaneous team member stress metrics via the data communication device;
analyze voice patterns to identify breaks in speak and responsiveness between team members; and
determine a team engagement metric based on the user stress metric, one or more contemporaneous team member stress metrics, and responsiveness between team members.

2. The computer apparatus of Claim 1, further comprising one or more physiological data recording devices (110) in data communication with the at least one processor, wherein:
the processor executable code further configures the at least one processor to:
receive physiological data from the one or more physiological data recording devices; and
correlate the physiological data with the audio / video stream; and
creating the user stress metric includes reference to the physiological data.

3. The computer apparatus of Claim 2, wherein:
the processor executable code further configures the at least one processor to:
identify a disposition of information for each team member; and
correlate a gaze estimate to the disposition of information to determine if team members are focused on a common data set; and
creating the user stress metric includes reference to the gaze estimate.

4. The computer apparatus of any preceding Claim, wherein the processor executable code further configures the at least one processor to:
determine a priority associated with each of the user stress metric and one or more contemporaneous team member stress metrics; and
weight the user stress metric and one or more contemporaneous team member stress metrics according to the associated priority when determining the team engagement metric.

5. The computer apparatus of any preceding Claim, further comprising a display (114), wherein the processor executable code further configures the at least one processor to:
receive at least one audio / video stream from a team member via the data communication device;
display the at least one audio / video stream from the team member on the display; and
determine the user stress with reference to the at least one audio / video stream from the team member.

6. The computer apparatus of any preceding Claim, wherein the processor executable code further configures the at least one processor as a machine learning neural network.

7. A method comprising:
receiving an audio / video stream from at least one audio / video sensor (108);
determining a user stress metric based on the audio / video stream;
receiving one or more contemporaneous team member stress metrics via a data link;
analyzing voice patterns to identify breaks in speak and responsiveness between team members; and
determining a team engagement metric based on the user stress metric, one or more contemporaneous team member stress metrics, and responsiveness between team members.

8. The method of Claim 7, further comprising:
receiving physiological data from one or more physiological data recording devices; and
correlating the physiological data with the audio / video stream,
wherein creating the user stress metric includes reference to the physiological data.

9. The method of Claim 8, further comprising:
identifying a disposition of information for each team member; and
correlating a gaze estimate to the disposition of information to determine if team members are focused on a common data set,
wherein creating the user stress metric includes reference to the gaze estimate.

10. The method of Claim 7, 8 or 9, further comprising:
determining a priority associated with each of the user stress metric and one or more contemporaneous team member stress metrics; and
weighting the user stress metric and one or more contemporaneous team member stress metrics according to the associated priority when determining the team engagement metric.

11. The method of any of Claims 7 to 10, further comprising:
receiving at least one audio / video stream from a team member;
displaying the at least one audio / video stream from the team member on a display; and
determining the user stress with reference to the at least one audio / video stream from the team member.

12. The method of any of Claims 7 to 11, further comprising recording the team engagement metric, user stress metric, and one or more contemporaneous team member stress metrics associated with each of a plurality of discreet tasks over time.

13. The method of Claim 12, further comprising determining a team composition based on the team engagement metric, user stress metric, and one or more contemporaneous team member stress metrics based on individual stress metrics during discreet tasks.

14. A team monitoring system comprising:
a plurality of team member monitoring computers, each comprising a computer apparatus as claimed in any of claims 1 to 6.
